(19) 【Europäisches Patentamt / European Patent Office / Office européen des brevets】

(11) **EP 4 511 642 B1**

(12) # EUROPEAN PATENT SPECIFICATION

(45) Date of publication and mention
of the grant of the patent:
**29.04.2026  Bulletin 2026/18**

(21) Application number: **23720099.3**

(22) Date of filing: **18.04.2023**

(51) International Patent Classification (IPC):
**G01N 23/046** (2018.01)  **A61B 6/03** (2006.01)
**A61B 6/00** (2024.01)  **H01J 35/14** (2006.01)

(52) Cooperative Patent Classification (CPC):
**G01N 23/046; A61B 6/06; A61B 6/4021;
A61B 6/5235; A61B 6/54; G21K 7/00;**
G01N 2223/204; G01N 2223/302; G21K 2207/00

(86) International application number:
**PCT/EP2023/060002**

(87) International publication number:
**WO 2023/203023 (26.10.2023 Gazette 2023/43)**

(54) **SECONDARY EMISSION COMPENSATION IN X-RAY SOURCES**

SEKUNDÄREMISSIONSKOMPENSATION IN RÖNTGENQUELLEN

COMPENSATION D'ÉMISSION SECONDAIRE DANS DES SOURCES DE RAYONS X

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.04.2022  EP 22169377**

(43) Date of publication of application:
**26.02.2025  Bulletin 2025/09**

(73) Proprietor: **Excillum AB
164 40 Kista (SE)**

(72) Inventors:
• **NILSSON, Daniel
164 40 KISTA (SE)**
• **HANSSON, Björn
164 40 KISTA (SE)**
• **LUNDSTRÖM, Ulf
164 40 KISTA (SE)**

(74) Representative: **AWA Sweden AB
Box 45086
104 30 Stockholm (SE)**

(56) References cited:
**US-A- 4 637 040      US-A1- 2021 137 469
US-A1- 2021 244 374**

# Description

Field

The present disclosure relates to a method for X-ray imaging, and to a related X-ray imaging system.

Background

It is well known in the field that secondary radiation can cause problems in X-ray sources, see e.g. US 2011/0176663. Secondary radiation is generally understood as any radiation other than the desired radiation and can originate, for example, from scattering of X-rays or from unintended electron beam interactions in the system. Typically, efforts are directed to reducing the amount of secondary radiation generated, which may be accomplished by mechanical design, e.g. to avoid direct line of sight from elements that may generate secondary radiation. Reduction of such secondary radiation may also be achieved by careful material selection, e.g. by providing elements that could otherwise generate secondary radiation with a coating of a low X-ray yield material (e.g. carbon). Furthermore, commonly used X-ray detector calibration procedures may, at least to some degree, reduce the influence of secondary radiation.

Further, a procedure known as *flat field correction* typically comprises exposing the detector to uniform radiation. Nominally this would result in equal readout from each pixel on the detector. However, various imperfections will result in variations between pixels. To compensate for this the values recorded during uniform illumination may be used to adjust subsequently recorded images. However, if the assumed uniform illumination comprises contribution from secondary radiation sources, i.e. it is not actually uniform, this procedure may reduce the influence of secondary radiation during imaging. A problem with this approach is that when a sample is present between the X-ray source and the detector the secondary radiation will, at least partly, be absorbed by the sample. Thus, since the assumption made of uniform illumination during the flat field calibration is not valid, the compensation may, when applied to an image comprising a sample, fail to eliminate the effects of secondary radiation.

A somewhat similar problem is related to scattered X-ray radiation making its way to the detector. This may particularly be an issue when doing CT scans. A way to compensate for this, proposed in EP3939510, is to calculate a simulated image created by the scattered radiation and subtract this simulated image from the acquired image to obtain a scatter reduced image. For this type of application, it is not evident how one should go about to actually obtain an image created only from the scattered radiation, hence a simulation was used.

In US 2005/0123097 there is disclosed a method where two images of the same object are acquired with different X-ray energy spectra. The two images are subtracted to enhance certain features, e.g. subtracting unwanted images of fatty tissue in a mammogram from images of potential cancer lesions or removing bone images from chest X-ray images. The two images are created by providing a multi-material target and directing the electron beam to different parts of the target to obtain different X-ray energy spectra. Embodiments where the different target materials are provided as layers on top of each other, sometimes provided on a substrate transparent to X-rays, are also disclosed.

US 2021/244374 A1 discloses prior art according to the preamble of claim 1.

Summary

The present disclosure proposes a way to compensate for undesired secondary X-ray radiation that may be created in an X-ray source by electron beam interaction with, for example, an aperture (a beam-limiting element for the electron beam) and/or a target substrate. Such secondary emission of X-ray radiation adversely affects the image quality that can be obtained when using the X-ray source for illumination. The proposed solution involves recording two images, one where the electron beam is directed to the target and one where the beam is directed in such a way that only the secondary radiation is emitted. The final image is then formed by generating a difference image between the two images.

In general, it would be preferable to eliminate all secondary radiation, i.e. in an ideal case all emitted X-ray radiation comes from interaction between the focused electron beam and the target at a desired location. However, in practice there will always be some secondary radiation generated elsewhere in the X-ray source. Secondary radiation may be viewed as having two components, affecting the generated X-ray images somewhat differently, as discussed below.

One type of secondary radiation is generated some distance upstream of the target (as seen along the electron beam). This type of secondary radiation will typically manifest as a well-defined spot in the final image. If a set of images are acquired with the object at different rotation angles and these images are used to make a three-dimensional reconstruction of the object, this type of secondary radiation may cause ring-like features in the reconstruction. In a sense, the image is created from two well-defined X-ray sources, one where the focused electron beam impacts the target and one where the electron beam interacts with some other structure upstream of the target. One way of isolating this type of secondary radiation is to blank out the electron beam, i.e. deflect the electron beam to a position away from the target where substantially no X-ray radiation is emitted from the X-ray source. Provided that the structure where the secondary radiation is created is located upstream of the beam deflector, the contribution to the final image from the secondary radiation will be the same when directing the electron beam towards the target as when the beam is blanked, and the effect thereof in the final image can be eliminated by generating a difference image between two images (blanked and non-blanked electron beam).

Another type of secondary radiation is generated close to the target, e.g. by interaction between the electron beam and a target substrate or by interaction between electrons scattered from target and a backscatter collector. This type of radiation will typically manifest as a general blurring of the resulting X-ray image. Since the secondary radiation is generated close to the exit window it may be emitted over a large angular range, thereby in a sense making the effective X-ray spot larger and less well defined. To be able to isolate the contribution from this type of secondary radiation, the electron beam must impact the target and/or the target substrate under conditions such that the X-ray radiation created by interaction between the electron beam and the target is not emitted from the source.

One example of a source of secondary radiation is apertures or other beam-limiting elements in the electron beam path. The purpose of having an aperture that geometrically limits the electron beam is to have a well-defined electron beam size at some point and/or to limit the angles at which electrons propagate further in the system. The dimension of the aperture is determined inter alia by requirements on electron beam spot size at the target and the total electron beam throughput. However, electrons not passing through the aperture will interact with the material defining the aperture (i.e. with the beam-limiting element) and at least to some extent cause X-ray radiation to be generated. To avoid this, the material defining the aperture may be made of, or at least be coated with, a material with low X-ray generation efficiency. An alternative may be to provide the aperture as a hole in a relatively thick and dense material, such that X-ray radiation generated by electrons impacting on this material is absorbed (e.g. reabsorbed in the material) and does not reach the exit window. It may be difficult to eliminate the secondary radiation entirely using these approaches, but at least some reduction may be achieved. Another approach may be to not have a direct line of sight between the aperture and the exit window through which X-ray radiation is emitted from the source. The latter option may require some added complexity in some cases, e.g. in transmission configurations where X-rays are by definition emitted in the same direction as the electron beam. Furthermore, downstream from the aperture there should not be any other objects than the target with which the electron beam can interact to generate X-ray radiation that may exit the source.

A further potential source of secondary radiation is interaction between electrons backscattered from the target and surrounding parts of the X-ray source. It is known in the art to provide a backscatter collector (or trap) to capture the scattered electrons and the energy they carry. Interaction between the backscatter collector or some other part of the X-ray source and the electrons may in turn generate secondary radiation that can be emitted through the exit window. To eliminate, or at least reduce, this contribution to the emitted radiation, surface treatments (see WO 2020/052773) and/or geometric constraints (see US 2012/0170715) may be applied.

In cases where the X-ray source comprises electron optics for focusing and deflecting the electron beam, and in cases wherein the target comprises parts where a low X-ray yield substrate is exposed, the inventive principles disclosed herein may be implemented by taking a first image with the electron beam directed to an intended working position, i.e. on a target layer for instance comprising tungsten (W), and taking a second image with the electron beam directed to a location where the substrate, e.g. comprising diamond, is exposed (i.e. where there is no target layer on the substrate). By moving the sample so that it is imaged at the same location on the detector for each image, the image generated by X-rays emanating from the target layer may be extracted by generating a difference image between the images (e.g. by subtracting corresponding pixel values of the two images). Provided the shift in image position on the detector is small compared to the pixel resolution the sample may not need to be moved. Secondary radiation generated from some other structure, e.g. material defining an aperture, in the X-ray source will be present in both images and thus eliminated when generating the difference image.

Provided that electron beam steering and shaping is performed downstream (relative to the electron beam propagation direction) from the structures generating the secondary radiation, the contribution from this secondary radiation will be the same in the two images.

In other embodiments, the electron beam may be directed in the same way when recording the first and second images while instead the target is moved between image acquisitions so that the first image is exposed with the electron beam directed to the target layer and the second image is recorded with the electron beam directed so that only secondary radiation contributes to the image. In this way there is no need to move the sample between the images since the relative orientation between electron beam, sample, and detector is the same for the two images. The X-ray source in this case comprises an actuator arranged to move the target with sufficient precision, typically the order of $\mu$m, in a vacuum tight arrangement.

X-ray radiation generated by electrons penetrating the target layer, i.e. X-ray radiation generated from electron beam interaction with the substrate supporting the target layer rather than the target layer itself, will create an image that is substantially the same as the one generated by electrons impacting directly on the substrate, i.e. on a bare portion of the substrate, provided that scattering from the target layer may be neglected.

In cases where negligible secondary radiation is generated by interactions between the electron beam and structures located upstream beam deflection elements a first image may be acquired with the electron beam directed towards the target layer and a second image may be acquired with the electron beam directed towards an exposed surface of the target substrate. To compensate for a shift of image position on the detector the sample may be moved accordingly, or the acquired images may be shifted (aligned) so that they overlap prior to creating a difference image. Since there is negligible secondary radiation that is independent on where the electron beam impacts on the target no noticeable artefacts will be created by aligning the images.

In cases where a substantial fraction of the incoming electrons is absorbed and/or scattered in the target layer, a more elaborate procedure may be required. By deflecting the electron beam to a location from which no generated X-ray radiation can exit the X-ray source or at least not reach the detector, e.g. a shielded electron dump, an image created only from secondary radiation generated from other structures (e.g. the beam-limiting element for the electron beam) within the X-ray source may be obtained. The electron dump may be arranged as any part of the source that can withstand the thermal load imposed by the electron beam and wherein any X-ray radiation generated by the impacting electron beam does not reach the detector. By generating a difference image (e.g. by subtracting pixel values of this image from corresponding pixel values of the image acquired when the electron beam is directed towards the target layer), the influence of the secondary radiation generated at the beam limiting element may be eliminated or at least substantially reduced. To also eliminate the influence of X-ray radiation generated in the substrate, an image may be acquired with the electron beam directed towards the substrate but away from the target layer and with the sample moved so that it is imaged at the same position on the detector as when the electron beam is directed towards the target layer. The contribution from secondary radiation generated at the beam-limiting element may be eliminated also from this image by subtraction of the image acquired with the electron beam directed to the electron dump. The resulting image may then be scaled to compensate for absorption and/or scattering in the target layer. After scaling, pixel values of the image may be subtracted from corresponding pixel values of the difference image generated between the image recorded with the electron beam directed towards the target layer and the image generated with the electron beam directed to the electron dump. The scale factor may be known from target design or may be determined during a calibration procedure. As will be understood, a suitable target thus comprises a substrate that is only partly covered with a target layer (e.g. made from tungsten), while some parts of the substrate lack such target layer.

In the discussion above, it has been assumed that the images associated with secondary radiation generated at the aperture (beam-limiting element) may be considered identical irrespective of sample movement. In most practical cases this will be an acceptable assumption. In case it is not, i.e. when an image of the sample created by the secondary radiation changes considerably upon sample movement, a more elaborate method may be employed. A total of four images may be recorded. A first image is then taken with the sample in a first position and with the electron beam directed towards the target. A second image is taken with the sample still in the first position and with the electron beam directed so that only the secondary radiation from the aperture reaches the detector. A third image is taken with the electron beam deflected towards a part of the target where the substrate is exposed, and the sample is moved to a second position so that the image of the sample is at the same location as for the first image. A fourth image is taken with the sample in the second position and with the electron beam directed as for the second image. By subtracting the second image from the first image to generate a fifth image and subtracting the fourth image from third image to generate a sixth image, respectively, the effect from secondary radiation generated at the aperture is eliminated (or at least reduced). Then, by subtracting the sixth image, suitably scaled to compensate for scattering and/or absorption in the target layer, from the fifth image the effect from secondary radiation generated by electrons interacting with the substrate is also eliminated. As discussed above, subtracting an image from another means that a difference image between the two images is generated, e.g. by subtracting pixel values of one image from corresponding pixel values of the other image. For example, a difference image between the first and the second image can be generated by subtracting pixel values of the second image from corresponding pixel values of the first image. Pixel values may in some embodiments be scaled prior to subtraction.

For X-ray sources where secondary radiation created by electrons backscattered from the target make a substantial contribution to the image formed on the detector it may be advantageous to provide regions on the target for which no X-ray radiation is emitted towards the sample and the detector when the electron beam is directed thereto. This may be accomplished by providing local X-ray shielding, e.g. on the outside of the target for a transmission target, or by providing a region of the target that has no line of sight to the exit window. By directing the electron beam to such a location, only X-ray radiation created by the backscattered electrons will reach the detector, thus forming an image that can be subtracted from an image acquired with the electron beam directed to the intended target location.

A corresponding procedure may be performed to eliminate contributions from backscattered electrons to images formed with the electron beam directed to the exposed substrate of a transmission target. Once the backscatter contribution has been eliminated the image acquired with the electron beam directed towards the substrate may, possibly after suitable scaling, be subtracted from the image acquired with the electron beam directed towards the target layer.

In embodiments where an image comprising substantially only X-ray radiation generated at some structure upstream of the electron beam deflector (e.g. at an aperture) the image may be thresholded before subtraction from another image. The thresholding may be performed so that only pixels that actually received secondary radiation are used in the subtraction while other pixel values are set to zero (i.e. in effect the corresponding pixels in the original image are left unchanged after subtraction). A suitable threshold may for example be found as a multiple of the standard deviation of the pixel values or as a fraction of the maximum pixel value in the image. This may be an advantageous procedure in that the amount of noise in the final image may be less than what would be the case if all pixel values were used when generating the difference image (i.e. in the subtraction procedure). Other means for reducing the noise may also be employed, such as local median filtering of pixel values or other well-known image processing algorithms.

The methods outlined above may be applied when doing CT scans. In particular, images with the electron beam blanked out may be recorded for at least some of the sample orientations used during the scan to compensate for secondary radiation generated at an aperture, as discussed above. It may be advantageous to acquire the compensation images for a subset of the sample orientations used for the entire scan, for example good reconstruction results have been obtained by acquiring a blanked-out image for every tenth sample orientation. Instead of blanking out the electron beam it may be directed to a part of the target where the substrate is exposed, in this way somewhat sharper images may be obtained after subtraction of the compensation images. However, by blanking out the electron beam the total X-ray dose applied to the sample may be smaller.

The different approaches described above may be combined in different ways depending on which contributions to the secondary emission that need to be considered.

Brief description of the drawings

In the detailed description below, reference will be made to the accompanying drawings, on which:

Figure 1 is a schematic overview of an X-ray system according to the invention.
Figure 2 illustrates how the electron beam may be directed to different locations on the target and how the sample may be moved accordingly.
Figure 3 illustrates schematically a method for X-ray imaging.

Detailed description

While the inventive ideas presented herein have been discussed and summarized above to provide a thorough understanding thereof, specific implementations will be described below to further illustrate how the invention can be put into practice.

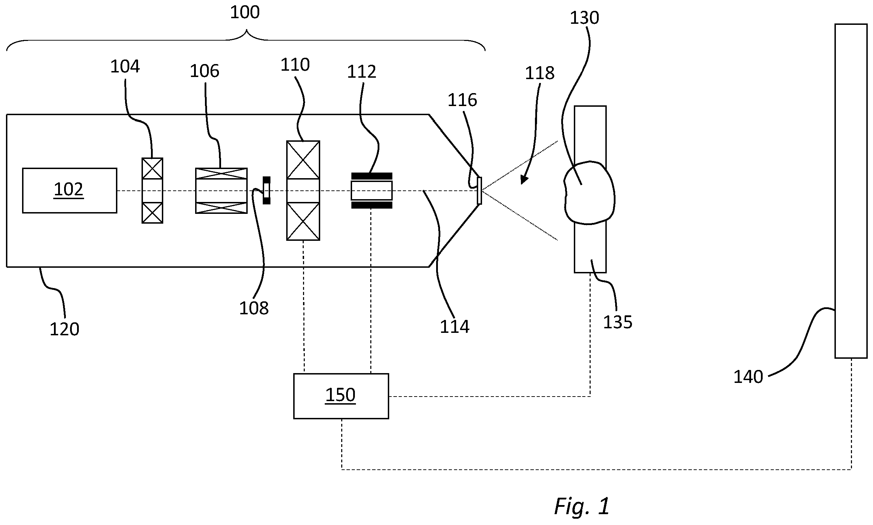

By way of introduction, Figure 1 schematically shows an exemplary imaging system comprising an X-ray source 100. The X-ray source as shown includes an electron gun 102 configured to form an electron beam 114. The electron beam emitted from the electron gun 102 is aligned and shaped using alignment coils 104 and stigmator coils 106, respectively, before reaching a beam-limiting element in the form of an aperture 108. As discussed in the summary above, such an aperture has the purpose of limiting the geometrical spread of the electron beam before it enters downstream (relative to the propagation direction of the electron beam) electron beam optics. The aperture 108 typically has a diameter, i.e. opening dimension, in the order of 1 mm. After having passed the aperture 108, the electron beam reaches electron optics, typically comprising one or more focusing lenses 110 and one or more deflectors 112. The electron beam optics is used for directing and focusing the electron beam to an intended location on a target 116. The target 116 typically comprises a high-Z (Z > 20) material that generates X-rays 118 upon electron impact. A typical target material in this type of X-ray source is tungsten (W), present as a film on a substrate material, e.g. made from diamond. These components of the X-ray source are enclosed within a low-pressure (vacuum) enclosure 120. The generated X-ray radiation 118 may be used for imaging a sample or an object 130, located at a sample position, using a detector 140. The X-ray source also includes a controller 150 that, according to the principles disclosed herein, is configured to record, using the detector 140, a first image with the electron beam directed to a first position, record a second image with the electron beam directed to a second position, and generate a difference image between the first and the second image, e.g. by subtracting pixel values of the second image from corresponding pixel values of the first image. The controller 150 may also be responsible for controlling the lenses 110 and the deflector 112 to direct the electron beam to said positions. As illustrated in Figure 1, the controller 150 may be operationally coupled to the electron optics 110, 112 and the detector 140.

Hence, an X-ray imaging system according to an embodiment comprises an X-ray source 100 as discussed above, a sample position (illustrated by the sample 130 in Figure 1), and a detector 140. The X-ray source 100 comprises an electron source 102 arranged to provide an electron beam, a target 116 arranged to produce X-ray radiation upon impact by the electron beam, means such as electron optics 110, 112 for directing the electron beam to a first position on the target and a second position (which may or may not be on the target), and a controller 150 arranged to record, using the detector, a first image with the electron beam directed to the first position, record a second image with the electron beam directed to the second position, and generate a difference image between the first and the second image. The imaging system may also comprise an actuator (not shown) arranged to move the target in relation to the electron beam, so that directing the electron beam to the first and second positions may involve steering/deflecting the electron beam using electron optics and/or moving the target. The imaging system may also comprise a manipulator for moving the X-ray source and the sample position in relation to each other. Such manipulator may, for example, be a translation stage 135 to which a sample can be mounted, as shown in Figure 1; other examples include putting the X-ray source on a translation stage or providing the X-ray source with feet that enable translation. Preferably, the manipulator is also operatively connected to the controller 150, as schematically illustrated by a dashed line for the translation stage 135 in Figure 1.

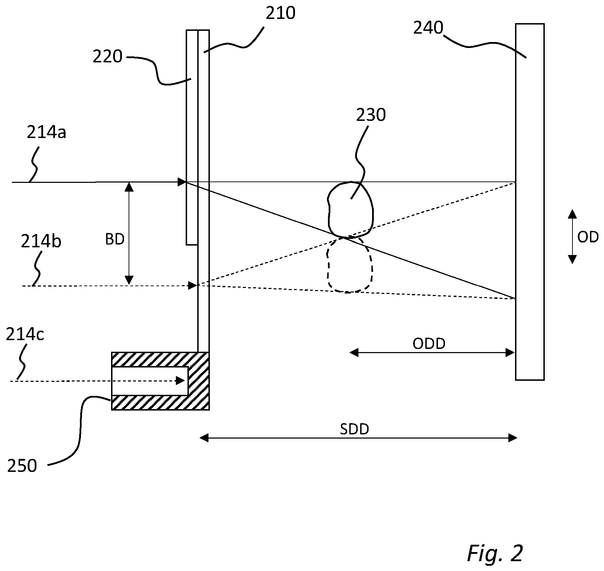

Figure 2 schematically shows the target 210, 220, the sample/object 230, and the detector 240 in more detail. According to the principles disclosed herein, influence of unwanted X-ray radiation on the captured image is reduced by subtracting one or more reference images from a main image. The unwanted X-ray radiation is generally referred herein to as secondary emission radiation. In order to capture the main image and the one or more reference images, the electron beam is moved (deflected) between different locations for capturing the various images involved. In Figure 2, three different electron beam locations are illustrated; a first location is shown at 214a, where the electron beam impacts a target layer 220 for intended generation of X-ray radiation; a second location is shown at 214b, where the electron beam does not impact the target layer 220 but instead directly impacts the underlying substrate 210; a third location is shown at 214c, where the electron beam impacts an electron dump 250. As will be understood, arrows 214a, 214b and 214c in Figure 2 represent the same electron beam directed at different locations. When the electron beam impacts directly upon the substrate 210, as shown at 214b, some X-ray radiation may be generated by interaction between the electron beam and the substrate material. It will be appreciated that X-ray radiation may also be generated by interaction between the electron beam and the substrate when the electron beam is directed to the target material 220, as shown at 214a, since some electrons may pass through the target material 220. On the other hand, when the electron beam impacts the electron dump 250, no X-ray radiation generated from interaction between the electron beam and the electron dump exits the X-ray source, or at least no X-ray radiation generated from interaction between the electron beam and the electron dump reaches the detector. Hence, when the electron beam impacts the electron dump 250, any X-ray radiation reaching the object 230 and the detector 240 is predominantly comprised of X-ray radiation generated at the aperture 108 located upstream from the target (see Fig. 1).

Regardless of whether the electron beam is directed to location 214a, 214b or 214c, a background of X-ray radiation generated at the aperture 108 will be present at the object 230 to be imaged. Such background can be reduced in the captured image by taking the difference between an image captured with the electron beam at location 214a and an image captured with the electron beam at location 214c. Taking a difference between images may for example involve taking a difference between each corresponding pixel values of the images.

When the electron beam is directed towards the substrate 210, as shown at 214b in Figure 2, the X-ray radiation reaching the object 230 may include both a contribution from radiation generated at the aperture 108 and a contribution generated in the substrate 210.

When an image captured with the electron beam at location 214b is involved, i.e. impacting directly upon the substrate 210, it may *á priori* be assumed that the object 230 must be moved correspondingly in order to allow a comparison with (subtraction from) an image captured with the electron beam directed towards the target material 220 as indicated at 214a since X-ray radiation is generated at two different locations. Figure 2 illustrates deflection of the electron beam 214a, b between points separated a distance of *BD* on the target. The electron beam is shown as a solid arrow 214a impacting a first point, and a dashed arrow 214b impacting a second point on the target. As discussed, the first point is located in a region where the electron beam 214a impacts the target layer 220 on the target, while the second point is located in a region where the electron beam 214b does not impact the target layer 220 but rather directly impacts the substrate 210. In order to obtain an image on the detector 240 at the same position for the two electron beam impact points, the sample or object 230 should be moved a distance *OD*, which may be calculated as

$$OD = BD\frac{ODD}{SDD}$$

where *ODD* is the distance from the sample to the detector (object detector distance) and *SDD* is the distance from the target to the detector (source-detector distance). The distance that the sample should be moved between image acquisitions may thus be calculated, by considering congruent triangles, to be the displacement of the electron beam on the target scaled by the ratio between the distance between the sample and the detector and the distance between the source and the detector. For many practical applications the distance between the source and the sample, corresponding to *SDD - ODD* as shown in Figure 2, is small compared to the distance *SDD* from the source to the detector, which means that *SDD* ≈ *ODD,* and the sample may thus be moved a distance *OD* substantially equal to the displacement *BD* of the electron beam without this having a noticeable difference on the final result. As an example, the distance *ODD* between the sample and the detector may be 100 times the distance (*SDD - ODD*) between the source and the sample. In such case the sample should in principle be moved 99% of the electron beam displacement. If beam displacement is of the order of 10 μm then the sample should be moved about 9.9 μm. The 0.1 μm difference may be neglected especially since a typical pixel resolution on the detector is in the range of 100 μm. Thus, considering the magnification of about 100 in this example, the 0.1 μm error would only correspond to one tenth of a pixel. Similar arguments may be applied when considering thicker samples. The two sides of a thick sample are at different distances from the detector and motion should thus in principle be scaled differently for the two sides, but in practice an average scaling may be used without noticeably distorting the image.

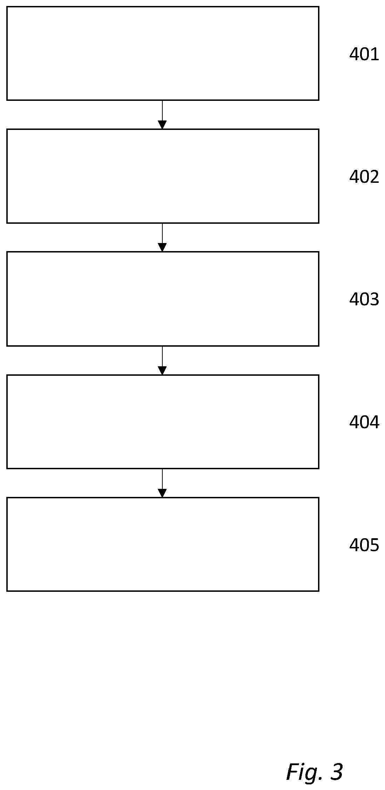

Figure 3 illustrates a method for X-ray imaging using an X-ray source comprising an electron source configured to provide an electron beam and a target configured to produce X-ray radiation upon impact by said electron beam. The method comprises directing 401 said electron beam to a first position on said target; recording 402, using a detector, a first X-ray image while directing said electron beam to said first position; directing 403 said electron beam to a second position; recording 404, using said detector, a second X-ray image while directing said electron beam to said second position; and generating 405 a difference image between the first image and the second image. As discussed above, for both the first and the second position of the electron beam, the X-ray radiation reaching the detector will comprise X-ray radiation generated by interaction between the electron beam and the beam-limiting element (the aperture), and any features or noise in the captured images can thus be reduced by generating such difference image between the two captured images.

To further reduce the effects caused by the secondary radiation, a scale factor may be determined from the respective exposure times used when capturing the first and the second images, and then scaling pixel values of the first and/or the second image before generating the difference image so that a difference in exposure time between the first and the second image may be compensated for.

To further improve the image quality of the final difference image, pixel values of the second image that are below a predetermined threshold may be set to zero before the difference image is generated.

Preferably, the method involves moving an object to be imaged so that a position of an image of the object on the detector is substantially the same for the first and the second images.

Depending on the circumstances, it may also be preferred to align the first and second images with each other using (*per se* known) image processing before generating the difference image.

The second position may correspond to a position on the target at which the electron beam impacts directly upon the target substrate. In such case, the method may further comprise directing the electron beam to a third position and capturing a third image, wherein the third position corresponds to the electron beam dump which is arranged so that substantially no X-ray radiation created by interaction between the electron beam and the electron beam dump is emitted from the X-ray source, at least no X-ray radiation that reaches the detector. A fourth image may then be created by subtracting pixel values of the third image from corresponding pixel values of the second image. The resulting pixel values of the fourth image can then be scaled by a scale factor, and the scaled fourth image as well as the third image can be subtracted from the first (main) image. In this way, secondary emission generated both at the aperture and in the target substrate is compensated for.

Arrangements, sources, and methods according to the invention may be used for different types of X-ray imaging such as X-ray microscopy, radiography, fluoroscopy, or CT scanning.

## Claims

1. An X-ray imaging system, comprising:

    - an X-ray source (100);
    - a sample position (130);
    - a detector (140) arranged to detect X-ray radiation downstream of said sample position (130); wherein said X-ray source (100) comprises:

        -- an electron source (102) arranged to provide an electron beam (114);
        -- a target (116) arranged to produce X-ray radiation (118) upon impact by said electron beam (114),

    **characterised in that** the X-ray source (100) is defined by:

        -- the target (116) comprising a substrate (210) and a target layer (220) at least partly covering said substrate (210), wherein said target layer (220) is arranged to produce X-ray radiation (118) upon impact by said electron beam (114);
        -- means for directing the electron beam (114) to a first position on said target layer (220) and a second position selected from a position on said target (116) at which the electron beam (114) impacts directly upon the substrate (210) and a position on an electron beam dump (250) arranged so that substantially no X-ray radiation created by interaction between the electron beam (114) and the electron beam dump (250) reaches the detector;
        -- a controller (150) arranged to

--- record, using said detector (140), a first image with the electron beam (114) directed to said first position, and a second image with the electron beam (114) directed to said second position, and
--- generate a difference image between the first image and the second image.

2. The system of claim 1, wherein said means for directing comprises a deflector (112) arranged to deflect the electron beam (114).

3. The system of claim 1 or 2, wherein said means for directing comprises an actuator arranged to move the target (116) in relation to the electron beam (114).

4. The system of any one of claims 1-3, further comprising a manipulator (135) for moving the X-ray source (100) and the sample position (130) in relation to each other.

5. The system of any one of claims 1-4, further comprising a beam-limiting element (108) arranged between the electron source (102) and the target (116).

6. The system of claim 5, wherein X-ray radiation (118) generated from interaction between the electron beam (114) and the beam-limiting element (108) reaches the detector (140) when the electron beam (114) is directed to the first position and the second position.

7. A method for X-ray imaging using an X-ray source (100) comprising an electron source (102) configured to provide an electron beam (114), and a target (116) comprising a substrate (210) and a target layer (220) at least partly covering the substrate (210), wherein the target layer (220) is arranged to produce X-ray radiation (118) upon impact by the electron beam (114), the method comprising:

   directing said electron beam (114) to a first position on said target layer (220);
   recording, using a detector (140), a first X-ray image while directing said electron beam (114) to said first position;
   directing said electron beam (114) to a second position;
   recording, using said detector (140), a second X-ray image while directing said electron beam (114) to said second position; and
   generating a first difference image between the first image and the second image;
   wherein, the second position is selected from a position on said target (116) at which the electron beam (114) impacts directly upon the substrate (210) and a position on an electron beam dump (250) arranged so that substantially no X-ray radiation created by interaction between the electron beam (114) and the electron beam dump (250) reaches the detector (140).

8. The method of claim 7, further comprising

   determining a scale factor from the exposure times of the first and second images, respectively; and
   scaling pixel values of at least one of the first image and the second image before generating the first difference image.

9. The method of claim 7 or 8, further comprising setting pixel values of the second image that are below a predetermined threshold to zero, and thereafter performing the step of generating the first difference image.

10. The method of any one of claims 7-9, further comprising: moving an object (230) to be imaged so that a position of an image of the object on the detector (140) is substantially the same for said first image and said second image.

11. The method of any one of claims 7-10, further comprising:
    aligning the first image and the second image before generating the first difference image.

12. The method of any one of claims 7-11, further comprising directing said electron beam (114) to a third position and recording a third image, wherein

    said first position corresponds to a position on the target (116) configured for X-ray production;
    said second position corresponds to an electron beam dump (250) arranged so that substantially no X-ray radiation created by interaction between the electron beam (114) and the electron beam dump (250) reaches the detector (140);
    said third position corresponds to a position on the target (116) not configured for X-ray production;
    generating a second difference image between the third image and the second image; scaling pixel values of the second difference image by a predetermined scale factor; and
    generating a third difference image between the first difference image and the scaled second difference image.

**Patentansprüche**

1. Röntgenbildgebungssystem, welches Folgendes umfasst:

- eine Röntgenquelle (100);
- eine Probenposition (130);
- einen Detektor (140), der zum Erkennen von Röntgenstrahlung stromabwärts der Probenposition (130) geeignet ist;

wobei die Röntgenquelle (100) Folgendes umfasst:

-- eine Elektronenquelle (102), die zum Bereitstellen eines Elektronenstrahls (114) geeignet ist;
-- ein Ziel (116), das zum Erzeugen von Röntgenstrahlung (118) beim Austreffen des Elektronenstrahls (114) geeignet ist,

**dadurch gekennzeichnet, dass** die Röntgenquelle (100) durch Folgendes definiert ist:

-- das Ziel (116), das ein Substrat (210) und eine Zielschicht (220), welche das Substrat (210) zumindest teilweise bedeckt, umfasst, wobei die Zielschicht (220) zum Erzeugen von Röntgenstrahlung (118) beim Auftreffen des Elektronenstrahls (114) geeignet ist;
-- Mittel zum Lenken des Elektronenstrahls (114) auf eine erste Position auf der Zielschicht (220) und eine zweite Position ausgewählt aus einer Position auf dem Ziel (116), an welcher der Elektronenstrahl (114) direkt auf das Substrat (210) auftrifft, und einer Position auf einer Elektronenstrahl-Auffangvorrichtung (250), welche derart angeordnet ist, dass im Wesentlichen keine Röntgenstrahlung, die durch eine Wechselwirkung zwischen dem Elektronenstrahl (114) und der Elektronenstrahl-Auffangvorrichtung (250) erzeugt wird, den Detektor erreicht;
-- eine Steuereinrichtung (150), die geeignet ist zum

--- Aufzeichnen, unter Verwendung des Detektors (140), eines ersten Bildes, wenn der Elektronenstrahl (114) auf die erste Position gelenkt wird, und eines zweiten Bildes, wenn der Elektronenstrahl (114) auf die zweite Position gelenkt wird, und
--- Erzeugen eines Differenzbildes zwischen dem ersten Bild und dem zweiten Bild.

2. System nach Anspruch 1, wobei das Mittel zum Lenken einen Deflektor (112) umfasst, der zum Ablenken des Elektronenstrahls (114) geeignet ist.

3. System nach Anspruch 1 oder 2, wobei das Mittel zum Lenken einen Aktuator umfasst, der zum Bewegen des Ziels (116) in Bezug auf den Elektronenstrahl (114) geeignet ist.

4. System nach einem der Ansprüche 1-3, welches ferner einen Manipulator (135) zum Bewegen der Röntgenquelle (100) und der Probenposition (130) in Bezug aufeinander umfasst.

5. System nach einem der Ansprüche 1-4, welches ferner ein Strahlbegrenzungselement (108) umfasst, welches zwischen der Elektronenquelle (102) und dem Ziel (116) angeordnet ist.

6. System nach Anspruch 5, wobei Röntgenstrahlung (118), die durch die Wechselwirkung zwischen dem Elektronenstrahl (114) und dem Strahlbegrenzungselement (108) erzeugt wird, den Detektor (140) erreicht, wenn der Elektronenstrahl (114) auf die erste Position und die zweite Position gelenkt wird.

7. Verfahren zur Röntgenbildgebung unter Verwendung einer Röntgenquelle (100), umfassend eine Elektronenquelle (102), die zum Bereitstellen eines Elektronenstrahls (114) konfiguriert ist, und ein Ziel (116), welches ein Substrat (210) und eine Zielschicht (220), die das Substrat (210) zumindest teilweise bedeckt, umfasst, wobei die Zielschicht (220) zum Erzeugen von Röntgenstrahlung (118) beim Auftreffen des Elektronenstrahls (114) geeignet ist, wobei das Verfahren Folgendes umfasst:

Lenken des Elektronenstrahls (114) auf eine erste Position auf der Zielschicht (220);
Aufzeichnen, unter Verwendung eines Detektors (140), eines ersten Röntgenbildes, während der Elektronenstrahl (114) auf die erste Position gelenkt wird;
Lenken des Elektronenstrahls (114) auf eine zweite Position;
Aufzeichnen, unter Verwendung des Detektors (140), eines zweiten Röntgenbildes, während der Elektronenstrahl (114) auf die zweite Position gelenkt wird; und
Erzeugen eines ersten Differenzbildes zwischen dem ersten Bild und dem zweiten Bild;
wobei die zweite Position ausgewählt ist aus einer Position auf dem Ziel (116), an welcher der Elektronenstrahl (114) direkt auf das Substrat (210) auftrifft, und einer Position auf einer Elektronenstrahl-Auffangvorrichtung (250), welche derart angeordnet ist, dass im Wesentlichen keine Röntgenstrahlung, die durch eine Wechselwirkung zwischen dem Elektronenstrahl (114) und der Elektronenstrahl-Auffangvorrichtung (250) erzeugt wird, den Detektor (140) erreicht.

8. Verfahren nach Anspruch 7, welches ferner Folgendes umfasst:

Bestimmen eines Skalierungsfaktors aus den Belichtungszeiten des ersten bzw. zweiten Bildes; und

Skalieren von Pixelwerten von mindestens einem aus dem ersten Bild und dem zweiten Bild vor dem Erzeugen des ersten Differenzbildes.

9. Verfahren nach Anspruch 7 oder 8, welches ferner Folgendes umfasst:

Einstellen von Pixelwerten des zweiten Bildes, die unterhalb einer vorbestimmten Schwelle liegen, auf Null und anschließendes Durchführen des Schrittes des Erzeugens des ersten Differenzbildes.

10. Verfahren nach einem der Ansprüche 7-9, welches ferner Folgendes umfasst:

Bewegen eines Objekts (230), das abgebildet werden soll, sodass eine Position eines Bildes des Objekts auf dem Detektor (140) im Wesentlichen die gleiche für das erste Bild und das zweite Bild ist.

11. Verfahren nach einem der Ansprüche 7-10, welches ferner Folgendes umfasst:

Ausrichten des ersten Bildes und des zweiten Bildes vor dem Erzeugen des ersten Differenzbildes.

12. Verfahren nach einem der Ansprüche 7-11, welches ferner Folgendes umfasst:

Lenken des Elektronenstrahls (114) auf eine dritte Position und Aufzeichnen eines dritten Bildes, wobei

die erste Position einer Position auf dem Ziel (116) entspricht, die zur Röntgenstrahlerzeugung konfiguriert ist;
die zweite Position einer Elektronenstrahl-Auffangvorrichtung (250) entspricht, die derart angeordnet ist, dass im Wesentlichen keine Röntgenstrahlung, die durch die Wechselwirkung zwischen dem Elektronenstrahl (114) und der Elektronenstrahl-Auffangvorrichtung (250) erzeugt wird, den Detektor (140) erreicht;
die dritte Position einer Position auf dem Ziel (116) entspricht, die nicht zur Röntgenstrahlerzeugung konfiguriert ist;

Erzeugen eines zweiten Differenzbildes zwischen dem dritten Bild und dem zweiten Bild;
Skalieren von Pixelwerten des zweiten Differenzbildes durch einen vorbestimmten Skalierungsfaktor; und
Erzeugen eines dritten Differenzbildes zwischen dem ersten Differenzbild und dem skalierten zweiten Differenzbild.

**Revendications**

1. Système d'imagerie par rayons X comprenant :

- une source de rayons X (100) ;
- une position d'échantillon (130) ;
- un détecteur (140) agencé pour détecter le rayonnement X en aval de ladite position d'échantillon (130) ; dans lequel ladite source de rayons X (100) comprend :

-- une source d'électrons (102) agencée pour fournir un faisceau d'électrons (114) ;
-- une cible (116) agencée pour produire un rayonnement X (118) lors de son impact par ledit faisceau d'électrons (114),

**caractérisé en ce que** la source de rayons X (100) est définie par :

-- la cible (116) comprenant un substrat (210) et une couche cible (220) recouvrant au moins partiellement ledit substrat (210), dans lequel ladite couche cible (220) est agencée pour produire un rayonnement X (118) lors de son impact par ledit faisceau d'électrons (114) ;
-- des moyens pour diriger le faisceau d'électrons (114) vers une première position sur ladite couche cible (220) et une deuxième position choisie parmi une position sur ladite cible (116) à laquelle le faisceau d'électrons (114) impacte directement le substrat (210) et une position sur un absorbeur de faisceau d'électrons (250) agencé de manière à ce qu'aucun rayonnement X, généré par l'interaction entre le faisceau d'électrons (114) et l'absorbeur de faisceau d'électrons (250) n'atteigne le détecteur ;
-- un contrôleur (150) agencé pour :

--- enregistrer, à l'aide dudit détecteur (140), une première image avec le faisceau d'électrons (114) dirigé vers ladite première position, et une deuxième image avec le faisceau d'électrons (114) dirigé vers ladite deuxième position ; et
--- générer une image de différence entre la première image et la deuxième image.

2. Système selon la revendication 1, dans lequel lesdits moyens de direction comprennent un déflecteur (112) agencé pour dévier le faisceau d'électrons (114).

3. Système selon la revendication 1 ou 2, dans lequel lesdits moyens de direction comprennent un actionneur agencé pour déplacer la cible (116) par rapport au faisceau d'électrons (114).

**4.** Système selon une quelconque des revendications 1 à 3, comprenant en outre un manipulateur (135) pour déplacer la source de rayons X (100) et de la position de l'échantillon (130) l'une par rapport à l'autre.

**5.** Système selon une quelconque des revendications 1 à 4, comprenant en outre un élément limiteur de faisceau (108) disposé entre la source d'électrons (102) et la cible (116).

**6.** Système selon la revendication 5, dans lequel un rayonnement X (118) généré par l'interaction entre le faisceau d'électrons (114) et l'élément limiteur de faisceau (108) atteint le détecteur (140) lorsque le faisceau d'électrons (114) est dirigé vers la première position et la deuxième position.

**7.** Procédé d'imagerie par rayons X utilisant une source de rayons X (100) comprenant une source d'électrons (102) configurée pour fournir un faisceau d'électrons (114), et une cible (116) comprenant un substrat (210) et une couche cible (220) recouvrant au moins partiellement le substrat (210), dans lequel la couche cible (220) est agencée pour produire un rayonnement X (118) lors de l'impact par le faisceau d'électrons (114), le procédé comprenant les étapes suivantes :

diriger ledit faisceau d'électrons (114) vers une première position sur ladite couche cible (220) ; enregistrer, à l'aide d'un détecteur (140), une première image radiographique tout en dirigeant ledit faisceau d'électrons (114) vers ladite première position ; diriger ledit faisceau d'électrons (114) vers une deuxième position ; enregistrer, à l'aide dudit détecteur (140), une deuxième image radiographique tout en dirigeant ledit faisceau d'électrons (114) vers ladite deuxième position ; et générer une première image de différence entre la première et la deuxième image ; dans lequel la deuxième position est choisie entre une position sur ladite cible (116) à laquelle le faisceau d'électrons (114) impacte directement le substrat (210) et une position sur un absorbeur de faisceau d'électrons (250) agencé de manière à ce qu'aucun rayonnement X, généré par l'interaction entre le faisceau d'électrons (114) et l'absorbeur de faisceau d'électrons (250) n'atteigne le détecteur (140).

**8.** Procédé selon la revendication 7, comprenant en outre :

la détermination d'un facteur d'échelle à partir des temps d'exposition des première et deuxième images, respectivement ; et la mise à l'échelle des valeurs de pixels d'au moins une de la première image et de la deuxième image avant la génération de la première image de différence.

**9.** Procédé selon les revendications 7 ou 8, comprenant en outre : la mise à zéro des valeurs de pixels de la deuxième image inférieures à un seuil prédéterminé, puis la génération de la première image de différence.

**10.** Procédé selon une quelconque des revendications 7 à 9, comprenant en outre : le déplacement d'un objet (230) à imager de sorte que la position de l'image de l'objet sur le détecteur (140) soit sensiblement la même pour ladite première image et ladite deuxième image.

**11.** Procédé selon une quelconque des revendications 7 à 10, comprenant en outre : l'alignement de la première image et de la deuxième image avant la génération de la première image de différence.

**12.** Procédé selon une quelconque des revendications 7 à 11, comprenant en outre : la direction dudit faisceau d'électrons (114) vers une troisième position et l'enregistrement d'une troisième image, dans lequel :

ladite première position correspond à une position sur la cible (116) configurée pour la production de rayons X ; ladite deuxième position correspond à un absorbeur de faisceau d'électrons (250) agencé de sorte qu'aucun rayonnement X, créé par l'interaction entre le faisceau d'électrons (114) et l'absorbeur de faisceau d'électrons (250), n'atteigne le détecteur (140) ; ladite troisième position correspond à une position sur la cible (116) non configurée pour la production de rayons X ; la génération d'une deuxième image de différence entre la troisième image et la deuxième image ; la mise à l'échelle des valeurs de pixels de la deuxième image de différence par un facteur d'échelle prédéterminé ; et la génération d'une troisième image de différence entre la première image de différence et la deuxième image de différence mise à l'échelle.

Fig. 1

*Fig. 2*

401

402

403

404

405

*Fig. 3*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110176663 A
- EP 3939510 A
- US 20050123097 A
- US 2021244374 A1
- WO 2020052773 A
- US 20120170715 A